# EUROPEAN PATENT APPLICATION

(11) **EP 4 585 045 A1**
(43) Date of publication of application: **16.07.2025**
(21) Application number: 23863174.1
(22) Date of filing: 05.09.2023
(51) Int. Cl.: A01N 1/02, A61P 27/02, A61J 3/00, A61L 27/38, A61K 35/545

(54) **TISSUE STORAGE CONTAINER AND TISSUE TRANSPLANT KIT**

(30) Priority: 06.09.2022 JP 2022141148
(71) Applicant: RACTHERA Co., Ltd., Tokyo 103-6012 (JP)
(72) Inventor: WATARI, Kenji, Kobe-shi, Hyogo 650-0047 (JP); NAKAGAWA, Takashi, Kobe-shi, Hyogo 650-0047 (JP); KUWAHARA, Atsushi, Kobe-shi, Hyogo 650-0047 (JP)
(74) Representative: Zimmermann & Partner Patentanwälte mbB
(86) International application number: PCT/JP2023/032341
(87) International publication number: WO 2024/053636

(57) **Abstract**

There is provided a tissue storage container excellent in visibility of minute pale white tissue such as a retina sheet and easy to handle. The tissue storage container 100 according to the present invention is a container for storing pale white tissue T having a volume of 0.01 to 5 mm³ immersed in a storage solution, and includes a body 1, a mounting portion 2, and a cap 3. The body 1 includes a cylindrical portion 11 and a tapered portion 12. The tapered portion includes a side 121 having a substantially circular cross section and a diameter decreasing downward, and a bottom 122 closing a lower end of the side. The bottom of the tapered portion is a flat bottom. In a vertical section including a central axis CL of the body, a side angle θ of the tapered portion is 105 to 115°. A diameter D of the bottom of the tapered portion is 2 to 4 mm. The cap is a screw cap or a crimp cap. Transparency of the body is 70% or more. A capacity of the body is 1.5 to 3.0 mL.

## Description

### [Technical Field]

The present invention relates to a tissue storage container for storing minute pale white tissue such as a retina sheet, and a tissue transplant kit including the tissue storage container. In particular, the present invention relates to a tissue storage container and a tissue transplant kit excellent in visibility of tissue and easy to handle.

### [Background Art]

Recently, for example, to treat ophthalmic diseases such as retinitis pigmentosa, it has been proposed to use three-dimensional retina derived from induced pluripotent stem cells (iPS cells) (for example, see Patent Literature 1).

As described in Patent Literature 1, the three-dimensional retina derived from iPS cells is obtained as a sphere-like cell aggregate by differentiation from the iPS cells to retina. A retina sheet obtained by cutting part of the cell aggregate will be transplanted to a recipient.

The retina sheet is cut by an operator in a Cell Processing Center (CPC), and then, for example, filled in a storage container such as a microtube for biochemical experiment for storage. Then, the storage container is shipped to a medical institution, and the retina sheet is taken out of the storage container by a hand of a medical personnel such as a doctor, and filled in a transplant device for transplantation.

However, since the retina sheet is pale white and similar in color to resin or the like that is a material for the storage container, and the retina sheet is minute tissue having a volume of about 0.01 to 5 mm³, it is not easy to see the retina sheet in the storage container.

Generally, since the retina sheet is filled in the storage container by the operator in a safety cabinet, it is difficult to check the storage container by bringing it close to the operator's eyes. In addition, to ensure sterility, the operator must cover the whole body before operation by wearing special clothes, goggles, and two layers of gloves. Thus, the operator needs to handle the storage container under a situation with poor visibility and difficulty in operation.

When the retina sheet is transplanted in a medical institution, a medical personnel involved in transplantation, who may not be skilled in handling of cells or tissue, will handle the retina sheet.

Thus, a storage container excellent in visibility of a retina sheet and easy to handle has been desired, but a storage container suitable for storing a retina sheet has not yet been proposed because the retina sheet itself is a new technology.

### [Citation List]

### [Patent Literature]

[Patent Literature 1]
WO2020/184720

### [Summary of Invention]

### [Technical Problem]

An object of the present invention is to provide a tissue storage container excellent in visibility of minute pale white tissue such as a retina sheet and easy to handle, and a tissue transplant kit including the tissue storage container.

### [Solution to Problem]

To solve the problem, the present inventors have diligently evaluated and studied visibility of tissue and ease of handling using commercial containers and prototype containers, and have found that a tissue storage container excellent in visibility of tissue and easy to handle is obtained by setting transparency of a container, a side angle of the container, a shape of a bottom of the container, or the like to specific ones, thereby completing the present invention.

That is, in order to achieve the object, the present invention provides a tissue storage container for storing pale white tissue having a volume of 0.01 to 5 mm³ immersed in a storage solution, comprising: a body having an opened upper end and a closed lower end; a mounting portion located above the body and having an opened upper end and an opened lower end communicating with the upper end of the body; and a cap mounted to the mounting portion and closing an opening at the upper end of the mounting portion, wherein the body includes a cylindrical portion having opened upper and lower ends, and a tapered portion located below the cylindrical portion and having an opened upper end communicating with the lower end of the cylindrical portion, the tapered portion includes a side having a substantially circular cross section and a diameter decreasing downward, and a bottom closing a lower end of the side, the bottom of the tapered portion is a flat bottom, in a vertical section including a central axis of the body, an angle of the side of the tapered portion to a direction perpendicular to the central axis of the body is 105 to 115°, a diameter of the bottom of the tapered portion is 2 to 4 mm, the cap is a screw cap or a crimp cap, transparency of the body is 70% or more, and a capacity of the body is 1.5 to 3.0 mL.

In the present invention, the "tissue" means a structure of a cell population having a structure in which one or more types of cells having different forms or natures are three-dimensionally arranged in a certain pattern. The "tissue" in the present invention includes "retinal tissue". The "retinal tissue" means tissue in which one or more types of retinal cells such as photoreceptor cells, horizontal cells, bipolar cells, amacrine cells, retinal ganglion cells, retinal pigment epithelial cells, progenitor cells thereof, or retinal progenitor cells constituting each retinal layer in living retina are three-dimensionally arranged to form a layer. As one aspect, one or more of the retinal cells described above may form a single layer or multiple layers in a certain pattern. The layers that may be included in the retinal tissue may be called a retinal pigment epithelial layer, an outer limiting membrane, a photoreceptor cell layer (outer granular layer), an outer reticular layer, an inner granular layer, an inner reticular layer, a ganglion cell layer, a nerve fiber layer, and an inner limiting membrane. The retina sheet cut from three-dimensional retina derived from iPS cells is also a type of retinal tissue. The tissue including the retina sheet is constituted by the plurality of cells as described above, and is generally pale white. Although the pale white tissue is not completely transparent, the tissue has a pale color and also has a minute volume of 0.01 to 5 mm³, and is thus not easily visible.

In addition, in the present invention, the "transparency" means transparency measured by a method described later.

In the present invention, the "upper end" means an end on an upper side when the tissue storage container is disposed such that the central axis of the body of the tissue storage container is along a vertical direction and the opening of the body closed by the cap is located on the upper side. "Above" means an upward direction when the tissue storage container is disposed as described above. The "lower end" means an end on a lower side when the tissue storage container is disposed as described above. "Below" means a downward direction when the tissue storage container is disposed as described above.

In the present invention, the "cross section" means a section cut along a plane perpendicular to the central axis of the body of the tissue storage container. The "vertical section" means a section cut along a plane along the central axis of the body of the tissue storage container.

Further, in the present invention, a numerical value range expressed by "lower limit value X to upper limit value Y" (X and Y are any numerical values) means a lower limit value X or more and an upper limit value Y or less.

According to the present invention, the transparency of the body of the tissue storage container is 70% (75%) or more, the angle of the side of the tapered portion of the body to the direction perpendicular to the central axis of the body is 105 to 115°, and the bottom of the tapered portion of the body is the flat bottom. Thus, even if the tissue stored in the tissue storage container is minute pale white tissue having the volume of 0.01 to 5 mm³, high visibility can be obtained. Since the capacity of the body is 1.5 to 3.0 mL and small, and the cap is the screw cap or the crimp cap having high sealability, the tissue storage container has high resistance to physical stress such as vibration during shipping or centrifugation, and is easy to handle.

Preferably, a length along the central axis of the body is 35 to 45 mm.

The present inventors have found that the preferred configuration as described above further facilitates handling of the tissue storage container.

Preferably, the body is made of at least one of materials including cycloolefin polymer (COP), polymethylpentene (TPX (registered trademark)), glass, polyethylene terephthalate (PET), and polystyrene (PS).

The preferred configuration described above provides advantages that the transparency of the body of 70% (75%) or more can be achieved, high resistance to physical stress is obtained, pH of the storage solution is easily kept constant, and the inside of the body is easily kept sterile.

Further, in order to achieve the object, the present invention provides a tissue transplant kit for transplanting tissue to a recipient, comprising: a container filled with pale white tissue having a volume of 0.01 to 5 mm³ and a storage solution; and an injector for transplanting the tissue to the recipient, wherein the container is the tissue storage container.

The injector included in the tissue transplant kit according to the present invention may include components such as a syringe, a tip, and a cannula, which may be assembled immediately before transplantation of tissue.

The tissue transplant kit according to the present invention may further include a cleaning solution or an administration solution, or the like.

Summarizing the above, the present invention relates to the following matters.
[1] A tissue storage container for storing pale white tissue having a volume of 0.01 to 5 mm³ immersed in a storage solution, comprising: a body having an opened upper end and a closed lower end; a mounting portion located above the body and having an opened upper end and an opened lower end communicating with the upper end of the body; and a cap mounted to the mounting portion and closing an opening at the upper end of the mounting portion, wherein the body includes a cylindrical portion having opened upper and lower ends, and a tapered portion located below the cylindrical portion and having an opened upper end communicating with the lower end of the cylindrical portion, the tapered portion includes a side having a substantially circular cross section and a diameter decreasing downward, and a bottom closing a lower end of the side, the bottom of the tapered portion is a flat bottom, in a vertical section including a central axis of the body, an angle of the side of the tapered portion to a direction perpendicular to the central axis of the body is 105 to 115°, a diameter of the bottom of the tapered portion is 2 to 4 mm, the cap is a screw cap or a crimp cap, transparency of the body is 70% or more, and a capacity of the body is 1.5 to 3.0 mL.
[2] The tissue storage container according to [1], wherein a length along the central axis of the body is 35 to 45 mm.
[3] The tissue storage container according to [1] or [2], wherein the body is made of at least one of materials including cycloolefin polymer, polymethylpentene, glass, polyethylene terephthalate, and polystyrene.
[4] A tissue transplant kit for transplanting tissue to a recipient, comprising: a container filled with pale white tissue having a volume of 0.01 to 5 mm³ and a storage solution; and an injector for transplanting the tissue to the recipient, wherein the container is the tissue storage container according to any one of [1] to [3].

### [Advantageous Effect of Invention]

The present invention can provide a tissue storage container excellent in visibility of minute pale white tissue such as a retina sheet and easy to handle, and a tissue transplant kit including the tissue storage container.

### [Brief Description of Drawings]

[Figure 1] Figure 1 is a schematic perspective view of an example of an outline configuration of a retina sheet.
[Figure 2] Figure 2 shows an example of a result of evaluation using microtubes for biochemical experiment.
[Figure 3] Figure 3 shows an example of a result of evaluation using sample cups for analyzer.
[Figure 4] Figure 4 shows prototype tissue storage containers produced using a 3D printer.
[Figure 5] Figure 5 is a vertical sectional view of an outline configuration of a tissue storage container according to one embodiment of the present invention.
[Figure 6] Figure 6 shows production examples of the tissue storage containers according to one embodiment of the present invention.
[Figure 7] Figure 7 shows an example of a result of evaluation using a microtube for biochemical experiment, sample cups for analyzer, and the tissue storage containers according to one embodiment of the present invention.

### [Description of Embodiment]

Now, with reference the accompanying drawings, a tissue storage container and a tissue transplant kit according to one embodiment of the present invention will be described by taking as an example a case where tissue stored in the tissue storage container is a retina sheet.

### <Retina sheet>

First, a retina sheet will be outlined.

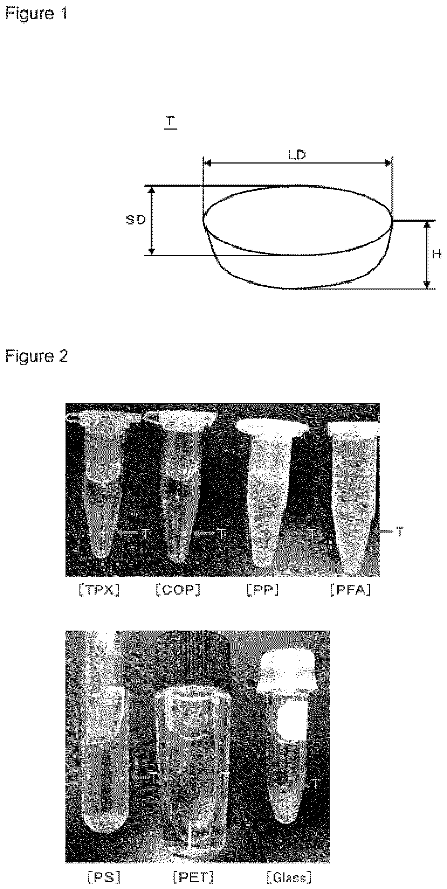

Figure 1 is a schematic perspective view of an example of an outline configuration of a retina sheet.

As described in Patent Literature 1, three-dimensional retina derived from iPS cells is produced as a sphere-like cell aggregate by differentiation from the iPS cells to retina. A retina sheet T is produced by cutting part of the cell aggregate.

As one aspect, a longer diameter LD of the retina sheet T is, for example, 300 to 3300 µm, preferably 600 to 2500 µm, more preferably 1100 to 1700 µm.

As one aspect, a shorter diameter SD of the retina sheet T is, for example, 100 to 2000 µm, preferably 200 to 1500 µm, more preferably 400 µm to 1100 µm.

As one aspect, a height H of the retina sheet T is, for example, 50 µm to 1500 µm, preferably 100 to 1000 µm, more preferably 200 to 700 µm.

As one aspect, a volume of the retina sheet T is, for example, 0.01 to 5 mm³, preferably 0.01 to 1.5 mm³, more preferably 0.07 to 0.57 mm³.

Details of the retina sheet T are the same as those described in Patent Literature 1, and further descriptions will be omitted here.

### <Process of study>

Next, before describing the tissue storage container according to this embodiment, a process of study to reach a configuration of the tissue storage container according to this embodiment will be described.

Figure 2 shows an example of a result of evaluation using microtubes for biochemical experiment.

As shown in Figure 2, a retina sheet T immersed in a storage solution was filled in each of 7 types of commercial microtubes for biochemical experiment, and visibility thereof was evaluated. Materials for the microtubes used were, from upper left, polymethylpentene (TPX(R)), cycloolefin polymer (COP), polypropylene (PP), fluororesin (PFA), polystyrene (PS), polyethylene terephthalate (PET), and glass. Each microtube has a round bottom (spherical bottom). Visibility was evaluated with the retina sheet T being in contact with a side of the microtube rather than the retina sheet T being settled to the bottom of the microtube.

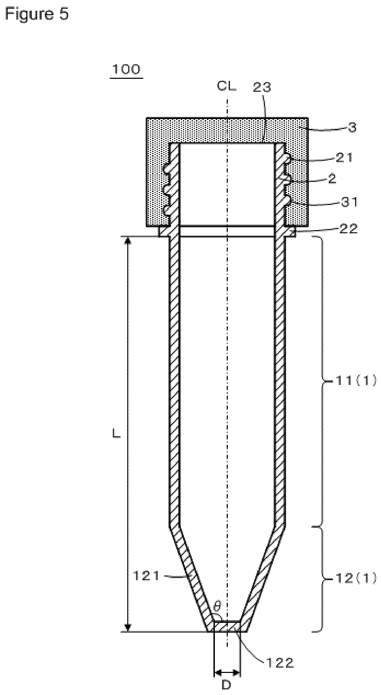

A side angle of each microtube (corresponding to an angle θ in Figure 5 described later) is as shown in Table 1 below.

**[Table 1]**

| Material | Side angle [° ] |
|---|---|
| TPX | 103 |
| COP | 100 |
| PP | 98 |
| PFA | 100 |
| FS | 130 |
| PET | 127 |
| Glass | 105 |

As shown in Figure 2, it was found that the retina sheet T was easily visible when the material for the microtube was cycloolefin polymer (COP), polymethylpentene (TPX(R)), glass, polyethylene terephthalate (PET), and polystyrene (PS).

The transparency of the microtube through which the retina sheet T was easily visible was as shown in Table 2 below, which was 70% (75%) or more.

**[Table 2]**

| Material | Transparency [%] |
|---|---|
| TPX | 76.5 |
| COP | 76.4 |
| PP | 43.6 |
| PFA | 36.9 |
| FS | 98.9 |
| PET | 91.8 |
| Glass | 79.6 |

Now, a method for measuring the transparency used in this embodiment will be described. The transparency was measured in such a manner that white A4 paper with a black line was placed on a stage of an upright microscope (manufactured by Zeiss), the microtube was placed on the A4 paper such that a longitudinal direction of the microtube was perpendicular to the black line, and the transparency was measured using an image captured by the upright microscope.

Specifically, for the black line that was used, a straight line having a width of 0.54 cm was drawn with PowerPoint (R) and printed out on the A4 paper by a printer. The transparency was calculated in such a manner that grayscale value on the black line and grayscale value on white background in the captured image were measured at a place with the microtube and a place without the microtube, and a difference between the grayscale value on the black line and the grayscale value on the white background at the place with the microtube was compared with that at the place without the microtube. More specifically, the difference between the grayscale value on the black line and the grayscale value on the white background at the place with the microtube was divided by the difference between the grayscale value on the black line and the grayscale value on the white background at the place without the microtube to calculate the transparency. The grayscale value was measured using "ImageJ" which is open-source image analysis software provided by National Institutes of Health.

As described above, the microtube typically has a round bottom. The round bottom has high resistance to an external force such as a centrifugal force during centrifugation, and is thus used as a bottom of the microtube. However, this is not designed in consideration of visibility of cells or tissue. The round bottom is less uniform in thickness than the side. In addition, the microtube is produced by injecting a material into an injection mold, and an inlet (gate) of the material is located on a central axis of the bottom, leaving a gate mark on the bottom. Thus, when the retina sheet T is settled to the bottom of the microtube or when the microtube is visually checked from the bottom, the minute retina sheet T is less visible in some cases.

Thus, visibility of the retina sheet T was evaluated using some commercial sample cups with flat bottom (planar bottom) for analyzer (containers without a cap).

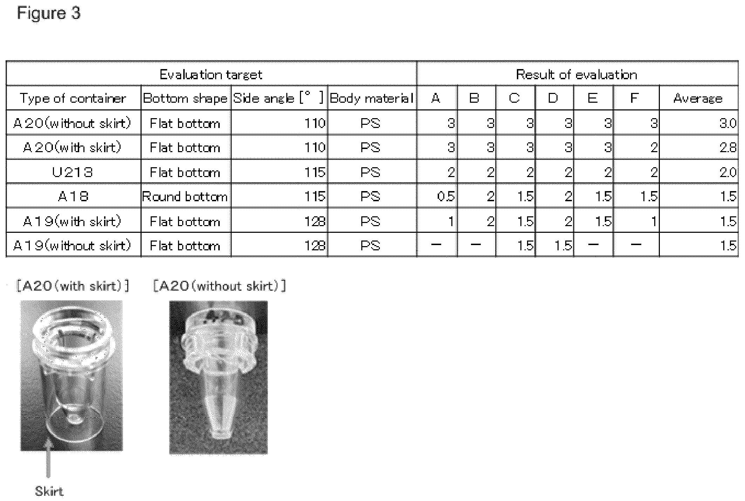

Figure 3 shows an example of a result of evaluation using sample cups for analyzer.

As shown in Figure 3, a retina sheet T immersed in a storage solution was filled in each of 6 types of commercial sample cups for analyzer with or without a skirt, and visibility thereof was evaluated. "A20" in Figure 3 is "Sample cup for automatic analysis A20 (capacity of 0.5 mL)" manufactured by AS ONE corporation. "U213" is "Sample cup U-213 (capacity of 1.2 cc) manufactured by MI CHEMICAL. "A18" is "Sample cup for automatic analysis A18 (capacity of 1.8 mL)" manufactured by AS ONE corporation. "A19 is "Sample cup for automatic analysis A19 (capacity of 1.5 mL)" manufactured by AS ONE corporation. A material for all the sample cups used was polystyrene (PS). Only "A18" had a round bottom, and the other sample cups each had a flat bottom. Visibility was evaluated with the retina sheet T being settled to the bottom of each sample cup.

The visibility of the retina sheet T was evaluated by 6 evaluators A to F scoring on the following scales.
"3": When the sample cup is laterally observed, the retina sheet T is immediately visible.
"2": When the sample cup is laterally observed, the retina sheet T is visible, but it takes a few seconds before becoming visible.
"1": When the sample cup is laterally observed, the retina sheet T is less visible, and the retina sheet T eventually becomes visible by changing a direction of the sample cup or the like.
"0": The retina sheet T is not visible even by changing the direction of the sample cup or the like.

In Figure 3, "1.5" means evaluation that it takes about a few tens of seconds before the retina sheet T becomes visible. "0.5" means evaluation that it takes about a few tens of seconds before the retina sheet T becomes visible even by changing the direction of the sample cup or the like.

As shown in Figure 3, it was found that the retina sheet T was more easily visible with the flat bottom than the round bottom. In addition, it was found that the retina sheet T was easily visible with the side angle (corresponding to an angle θ in Figure 5 described later) of 110 to 115°.

From the result in Figure 3, higher visibility is obtained with a smaller side angle, and only in terms of the side angle, the retina sheet T seems to be easily visible even with the side angle of 90 to 110°. On the other hand, the retina sheet T is smaller than a diameter of a cylindrical portion of the sample cup, and if the side angle is too small and close to 90° to increase a diameter and an area of the bottom, it may take labor to find the retina sheet T settled to the bottom. Thus, the side angle is desirably set to 105 to 115°, preferably 110 to 115°.

From the above result, the microtubes in Figure 2 and the sample cups "A20" and "U213" in Figure 3 were cut, upper parts of the cut microtubes and lower parts of the cut sample cups were bonded to produce prototype containers, and visibility of the retina sheet T was further evaluated.

Although details of the result of evaluation are omitted, for the prototype containers described above, it was confirmed that the retina sheet T was easily visible if the containers were each made of a material having transparency of 70% or more as described with reference to Figure 2, and had a flat bottom and a side angle of 105 to 115° as described with reference to Figure 3.

Further, the present inventors used a 3D printer to produce several types of prototype tissue storage containers having varied side angles and varied body lengths (corresponding to a length L in Figure 5 described later), and ease of handling thereof was evaluated.

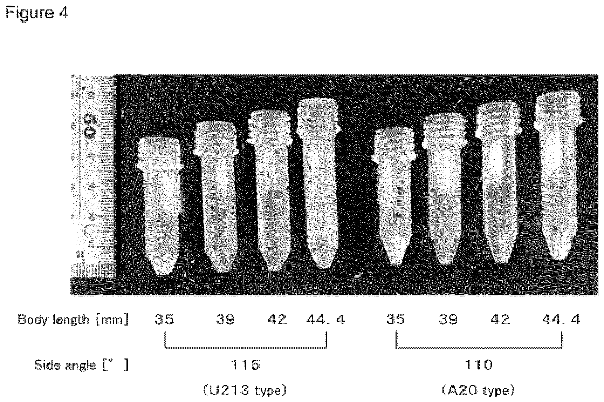

Figure 4 shows prototype tissue storage containers produced using a 3D printer.

Among the tissue storage containers in Figure 4, it was evaluated that one having a body length of 39 mm was easiest to handle, but for all the tissue storage containers, no problem was found in ease of handling. Thus, the body length is preferably 35 to 45 mm.

Assuming that an amount of a storage solution filled in the tissue storage container together with the retina sheet T is about 1.0 mL, the capacity of the body is preferably 1.5 to 3.0 mL.

As a cap for closing an opening of the tissue storage container, a screw cap or a crimp cap having high sealability is preferably used so as to increase resistance to physical stress such as vibration during shipping or centrifugation.

Further, in order for the retina sheet T to be immediately visible, the retina sheet T is desirably settled to a certain place. Thus, the diameter of the bottom of the tissue storage container is preferably set to be slightly larger than the longer diameter of the retina sheet T, and is preferably set to 2 to 4 mm.

### <Tissue storage container according to this embodiment>

Through the process of study as described above, the present inventors have reached the configuration of the tissue storage container according to this embodiment.

Figure 5 is a vertical sectional view of an outline configuration of the tissue storage container according to this embodiment.

As shown in Figure 5, the tissue storage container 100 according to this embodiment is for storing a pale white retina sheet T (not shown in Figure 5) having a volume of 0.01 to 5 mm³ immersed in a storage solution. The tissue storage container 100 includes a body 1 having an opened upper end and a closed lower end, a mounting portion 2 located above the body 1 and having an opened upper end and an opened lower end communicating with the upper end of the body 1, and a cap 3 mounted to the mounting portion 2 and closing an opening at the upper end of the mounting portion 2.

The body 1 includes a cylindrical portion 11 having opened upper and lower ends, and a tapered portion 12 located below the cylindrical portion 11 and having an opened upper end communicating with the lower end of the cylindrical portion 11.

The tapered portion 12 includes a side 121 having a substantially circular cross section and a diameter decreasing downward, and a bottom 122 closing a lower end of the side 121.

The bottom 122 of the tapered portion 12 is a flat bottom.

In a vertical section including a central axis CL of the body 1, an angle θ of the side 121 of the tapered portion 12 to a direction perpendicular to the central axis CL of the body 1 (lateral direction in Figure 5) is 105 to 115°, preferably 110 to 115°.

A diameter D of the bottom 122 of the tapered portion 12 (inner diameter of the bottom 122) is 2 to 4 mm.

The body 1 has transparency of 70% (75%) or more, and as a preferable aspect, the body 1 is made of at least one of materials including cycloolefin polymer, polymethylpentene, glass, polyethylene terephthalate, and polystyrene.

A capacity of the body 1 is 1.5 to 3.0 mL.

As a preferable aspect, a length L along the central axis CL of the body 1 is 35 to 45 mm.

The mounting portion 2 in this embodiment has an external thread 21 on its outer surface. To increase sealability of the cap 3, the lower end of the mounting portion 2 has a flange 22 against which a lower end of the cap 3 can abut.

In this embodiment, the body 1 and the mounting portion 2 are integrally made of the same material such as cycloolefin polymer (for example, "Daikyo Resin CZ" manufactured by DAIKYO SEIKO, LTD.), but the present invention is not limited to this. For example, the body 1 and the mounting portion 2 may be separately molded and then bonded together. In this case, the body 1 and the mounting portion 2 may be made of different materials. Since the cap 3 is mounted to the mounting portion 2, transparency of the mounting portion 2 has no influence on visibility of the retina sheet T. Thus, the transparency of the mounting portion 2 may not be necessarily 70% or more unlike the body 1.

The cap 3 in this embodiment is a screw cap.

Specifically, the cap 3 in Figure 5 is an outer cap having an internal thread 31 on its inner surface. The mounting portion 2 has the external thread 21 on its outer surface, and the external thread 21 of the mounting portion 2 and the internal thread of the cap 3 engage each other, so that the cap 3 is mounted to the mounting portion 2 to close the opening 23 at the upper end of the mounting portion 2.

The screw cap is not limited to that shown in Figure 5, but an inner cap having an external thread 21 and an internal thread 31 inverted, or a screw cap including an O ring may be used. A crimp cap may be also used as the cap 3 in this embodiment.

The tissue storage container 100 according to this embodiment as described above is excellent in visibility of a retina sheet T and easy to handle by setting the transparency of the body to 1 to 70% or more, setting the angle θ of the side 121 of the tapered portion 12 of the body 1 to 105 to 115° (preferably, 110 to 115°), and forming the bottom 122 of the tapered portion 12 of the body 1 into a flat bottom.

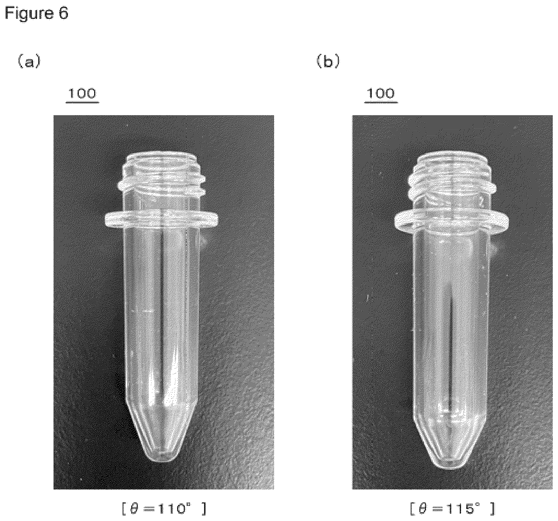

Figure 6 and Table 3 below show production examples of the tissue storage containers according to this embodiment as described above. The tissue storage container 100 in Figure 6(a) has an angle θ of the side 121 of 110°, and the tissue storage container 100 in Figure 6(b) has an angle θ of the side 121 of 115°. For both of the tissue storage containers 100, the body 1 is made of cycloolefin polymer (COP), the bottom 122 is a flat bottom, and the diameter D of the bottom 122 is 2.5 mm. For both of the tissue storage containers 100, the length L along the central axis CL of the body 1 is 39 mm, and the capacity of the body 1 is 2 mL. Further, although not shown, for both of the tissue storage containers 100, the cap 3 is a screw cap and a gate mark remains on the flange 22. As shown in Table 3, for both of the tissue storage containers 100, the transparency of the body 1 was 70% (75%) or more.

**[Table 3]**

| Material | Side angle *θ* [° ] | Transparency [%] | Corresponding figure |
|---|---|---|---|
| COP | 110 | 94.5 | Figure 6(a) |
| COP | 115 | 92.0 | Figure 6(b) |

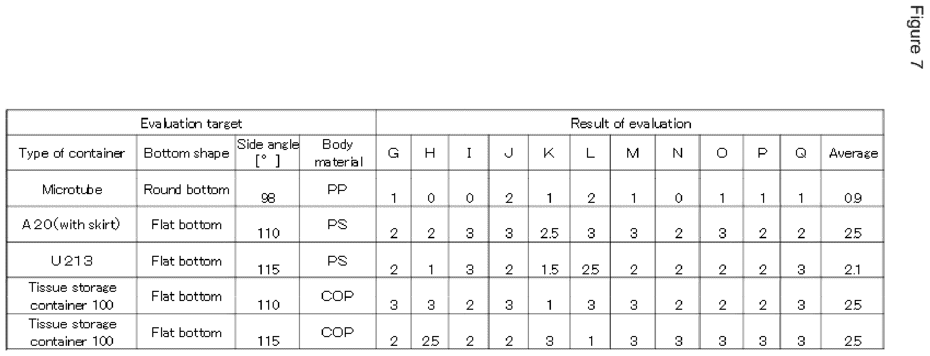

Figure 7 shows an example of a result of evaluation using a microtube for biochemical experiment, sample cups for analyzer, and the tissue storage containers 100 according to this embodiment.

As shown in Figure 7, a retina sheet T immersed in a storage solution was filled in each of a commercial microtube for biochemical experiment (made of polypropylene (PP)), two types of commercial sample cups for analyzer ("A20" and U213" described above), and the two types of tissue storage containers 100 shown in Figure 6 and Table 3 described above, and visibility thereof was evaluated. The visibility was evaluated with the retina sheet T being settled to the bottom of each container (microtube, sample cups, and tissue storage containers 100).

The visibility of the retina sheet T was evaluated by 11 evaluators G to Q scoring on the same scales as described with reference to Figure 3. The numerical value "2.5" which is found in the result of evaluation in Figure 7 but not found in that in Figure 3 means evaluation that when each container is laterally observed, the retina sheet T is immediately visible, but when observed from a certain direction, it takes about few seconds before the retina sheet T becomes visible. The other numerical values "0", "1", "1.5", "2", and "3" mean the same as in the case in Figure 3 ("sample cup" in the description of Figure 3 may be replaced by "each container").

As shown in Figure 7, it was found that for the tissue storage containers 100 according to this embodiment, the retina sheet T was easily visible at the same level as or a higher level than the commercial sample cups for analyzer.

### <Tissue transplant kit according to this embodiment>

The tissue transplant kit according to this embodiment is for transplanting tissue to a recipient, and includes a container filled with pale white tissue having a volume of 0.01 to 5 mm³ and a storage solution, and an injector for transplanting the tissue to the recipient. The container is the tissue storage container 100 as described above. The tissue transplant kit according to this embodiment may further include a cleaning solution or an administration solution, or the like.

The injector is not particularly limited as long as it is able to transplant tissue to the recipient. Typically, the injector includes a syringe filled with tissue and a storage solution, and a tip or a cannula for sucking and discharging the tissue and the storage solution. The injector may further include an instrument for assisting suction and discharge (for example, an injection kit or a tube (for example, "Ocular Irrigation Tube" REF# 169-30L-6 manufactured by EAGLE LABS)). The components of the injector such as the syringe and the tip may be already assembled or separated.

The storage solution is not particularly limited as long as it is a water solution in which tissue can be stored. For example, the storage solution may be a water solution including a buffer solution, an infusion solution, saline, water for injection, perfusate, or the like. There are many commercial solutions, and those skilled in the art can select an appropriate one depending on types of tissue or the like.

The administration solution is not particularly limited as long as it is a water solution that can be administered to a living body and has physical properties suitable for transplantation. For example, the administration solution may be a water solution including a buffer solution, an infusion solution, saline, water for injection, perfusate, or the like. The physical properties suitable for transplantation include pH, osmotic pressure, or the like, and typically, a neutral pH range or nearly isotonic osmotic pressure is selected. There are many commercial solutions, and those skilled in the art can select an appropriate one depending on types of tissue or the like.

The cleaning solution is not particularly limited as long as it is a water solution that can clean tissue. Typically, the water solution used as the storage solution or the administration solution is used.

In this embodiment, the case where the tissue stored in the tissue storage container 100 is the retina sheet T has been described. However, the tissue is not limited to the retina sheet T, but the tissue storage container 100 can store various types of pale white tissue having a volume of 0.01 to 5 mm³.

### [Reference Signs List]

- 1: body
- 2: mounting portion
- 11: cylindrical portion
- 12: tapered portion
- 121: side
- 122: bottom
- 100: tissue storage container
- CL: central axis of body 1
- D: diameter of bottom 122
- L: length along central axis CL of body 1
- T: retina sheet (tissue)
- θ: angle of side 121 to direction perpendicular to central axis CL

## Claims

1. A tissue storage container for storing pale white tissue having a volume of 0.01 to 5 mm³ immersed in a storage solution, comprising:
a body having an opened upper end and a closed lower end;
a mounting portion located above the body and having an opened upper end and an opened lower end communicating with the upper end of the body; and
a cap mounted to the mounting portion and closing an opening at the upper end of the mounting portion, wherein
the body includes a cylindrical portion having opened upper and lower ends, and a tapered portion located below the cylindrical portion and having an opened upper end communicating with the lower end of the cylindrical portion,
the tapered portion includes a side having a substantially circular cross section and a diameter decreasing downward, and a bottom closing a lower end of the side,
the bottom of the tapered portion is a flat bottom,
in a vertical section including a central axis of the body, an angle of the side of the tapered portion to a direction perpendicular to the central axis of the body is 105 to 115°,
a diameter of the bottom of the tapered portion is 2 to 4 mm,
the cap is a screw cap or a crimp cap,
transparency of the body is 70% or more, and
a capacity of the body is 1.5 to 3.0 mL.

2. The tissue storage container according to claim 1, wherein a length along the central axis of the body is 35 to 45 mm.

3. The tissue storage container according to claim 1 or 2, wherein the body is made of at least one of materials including cycloolefin polymer, polymethylpentene, glass, polyethylene terephthalate, and polystyrene.

4. A tissue transplant kit for transplanting tissue to a recipient, comprising:
a container filled with pale white tissue having a volume of 0.01 to 5 mm³ and a storage solution; and
an injector for transplanting the tissue to the recipient, wherein
the container is the tissue storage container according to claim 1 or 2.
